# EUROPEAN PATENT APPLICATION

(11) **EP 2 168 949 A1**
(43) Date of publication of application: **31.03.2010**
(21) Application number: 08775392.7
(22) Date of filing: 23.05.2008
(51) Int. Cl.: C07D 213/90, C12Q 1/68, G01N 33/53

(54) **METHOD FOR ELECTROCHEMICAL DETECTION OF SEQUENCES OF NUCLEIC ACIDS**

(30) Priority: 25.05.2007 ES 200701446
(71) Applicant: Universidad Autónoma de Madrid, 28049 Madrid (ES)
(72) Inventor: LORENZO ABAD, Encarnación, E-28049 Madrid (ES); PARIENTE ALONSO, Felix, E-28049 Madrid (ES); REVENGA PARRA, Monica, E-28049 Madrid (ES); GARCIA MENDIOLA, Tania, E-28049 Madrid (ES)
(74) Representative: Carpintero Lopez, Francisco
(86) International application number: PCT/ES2008/000364
(87) International publication number: WO 2008/145785

(57) **Abstract**

The present invention relates to a new compound derived from ruthenium, of formula (I), known as pentaamin ruthenium [3-(2-phenanthren-9-yl-vinyl)pyridine] complex, as well as the use thereof as an electrochemical indicator of hybridization in a method for the detection of hybridization between nucleic acid sequences, for the quantification of said sequences, as well as the detection of the presence of a mismatched base and the position thereof within a nucleic acid sequence.

## Description

### Field of the Invention

The present invention relates to a method for the detection of the hybridization of nucleic acids by means of electrochemical methods. More specifically, the present invention relates to a method for the detection of a certain DNA sequence, the presence of a mismatch in said sequence, as well as the position thereof, based on the use of a compound derived from ruthenium of general formula (I) as an indicator of hybridization in electrochemical biosensors.

### Background of the Invention

The molecular diagnosis based on the analysis of short DNA sequences offers sensitive and quantitative methods for the early detection of infectious diseases caused by pathogens (Garg, S. K. et al. Clin. Lab. Anal. 2003, 17(5), 155-163; Vernet, G. Virus Res. 2002, 82(1-2), 65-71).

The hybridization of DNA is the most common process for analyzing and detecting a particular gene or a segment of a nucleic acid. Among the methods based on hybridization are those based on surface plasmon resonances (Mullet, W. M. et al. Methods 2000, 22, 77-91*;* Sawata, S. et al. Biosens. Bioelectron. 1999, 14, 397-404*;* Livache, T. et al. Synth. Met. 2001, 121, 1443-1444*;* Wood, S. J. Microchem. J. 1993, 47, 330-337), fluorescence *(*Piunno, P. A. E. et al. Anal. Chim. Acta, 1994, 288, 205-214*;* Fodor, S. et al. Science, 1991, 251, 767-773*),* gravimetry *(*Okahata, Y. et al. Am. Chem. Soc. 1992, 114, 8299-8300*;* Minnuni, M. et al. Anal. Chim. Acta, 2003, 481, 55-64*)* or labeling with radioisotopes (Séller, G. H. et al. DNA Probes, 2nd ed. Stockton Press: New York, 1993; pp 149-213). The most used method is the use of radioactively labeled DNA or RNA fragments, known as probes. In fact, this methodology is the basis of controlled DNA amplification known as PCR (Polymerase Chain Reaction) which is used for DNA cloning and sequencing.

Among the methods based on hybridization, DNA biosensors have advantages in comparison with the mentioned methods. One of the advantages of these methods is the possibility of developing simple portable devices for this type of diagnosis. Furthermore, the coupling between these DNA biosensors and PCR amplification techniques can provide a high sensitivity for the detection of nucleic acid sequences (Meric, B. et al. Talanta 2002, 56(5), 837-846). The use of oligonucleotides labeled with fluorescence immobilized on a surface has allowed the development of high-density DNA arrays for the analysis of specific DNA sequences and of gene expression. However, these methods require the labeling of the target DNA *(*Berre, V. L. et al. Nucleic Acids Res. 2003, 31, e88*;* Dolan, P. L. et al. Nucleic Acids Res. 2001, 29, 107).

Since DNA has electroactive nucleic bands, electrochemical detection systems have also been developed by taking advantage of this property to directly detect the hybridized DNA, without having to make a marker intervene (Park, S. et al. Science, 2002, 295, 1503-1506*;* Drummond, T.G. et al. Nat. Biotech., 2003, 21, 1192-1199). DNA is generally immobilized on an electrode, and the difference of electric current measured before and after the hybridization is related to the amount of DNA fixed on the electrode (WO93/20230). However, this direct detection without a marker is not very sensitive.

For the purpose of improving the sensitivity, different approaches using an electroactive probe molecule or an electroactive marker have been developed. Thus, Palanti et al. (1996, Analytical Letters, 29, pp. 2309-31*)* describe different electroactive compounds which can be associated with DNA and can thus be detected by oxidation-reduction applying a potential to the electrode. Transition metal complexes, antibiotics, acridine or benzamide dyes and other DNA-intercalating agents have thus been used.

Since these electroactive probes or markers have better oxidation-reduction properties than DNA, their use allows obtaining a higher signal-to-noise ratio and better sensitivity.

Compounds derived from salophen (Revenga Parra, M. et al. "Comprehensive study of the interactions between DNA and new Schiff base ligands containing quinone functional groups" Biosensors and Bioelectronics. Volume: 22; Pages 2675-2681*)* and Osmium complexes (Del Pozo, M. V. et al. Anal. Chem. 2005a.77 (8), 2550-2557) have also been used as electrochemical indicators. In document CA02524263 ruthenium complexes which are incorporated to the double-strand at open circuit are used as indicators of hybridization.

The authors of the present invention have now developed a method which allows improving the sensitivity of the electrochemical detection of nucleic acid sequences as a result of the use of a novel ruthenium complex, prepared "in situ", known as pentaamin ruthenium [3-(2-phenanthren-9-yl-vinyl)pyridine] complex (referred to as RuL), as an indicator of hybridization in electrochemical biosensors.

The RuL complex, formed by the binding between the complex pentaamin ruthenium (Ru) and the ligand 3-(2-phenanthren-9-yl-vinyl)pyridine (L), is completely novel, as well as the use thereof for this purpose.

The high sensitivity and specificity achieved with the developed method allows detecting and quantifying, without needing any type of labeling, not only a certain nucleic acid sequence, but also a single mismatch in a base of said sequence, as well as the position thereof within the specific nucleic acid sequence, entailing furthermore a much faster and more cost-effective detection system than classic PCRs.

### Brief Description of the Drawings

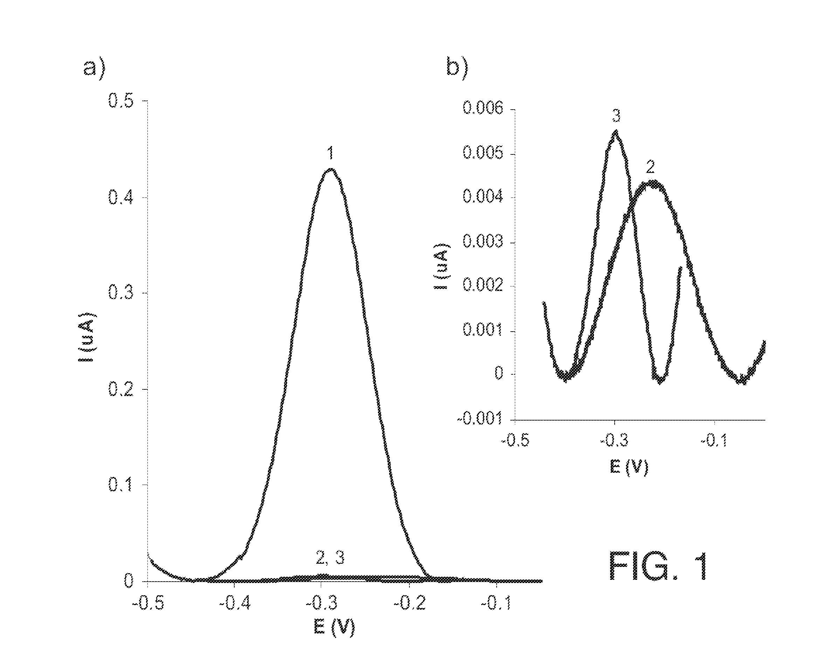

Figure 1. a) Differential pulse voltammograms (DPVs) of RuL accumulated in a gold electrode modified with SEQ ID NO. 1 before (2) and after the hybridization with the complementary strand (SEQ ID NO. 2) (1) and the non-complementary strand (SEQ ID NO. 3) (3). b) Amplification of the DPVs from -0.6 to 0 V of the potential scan.

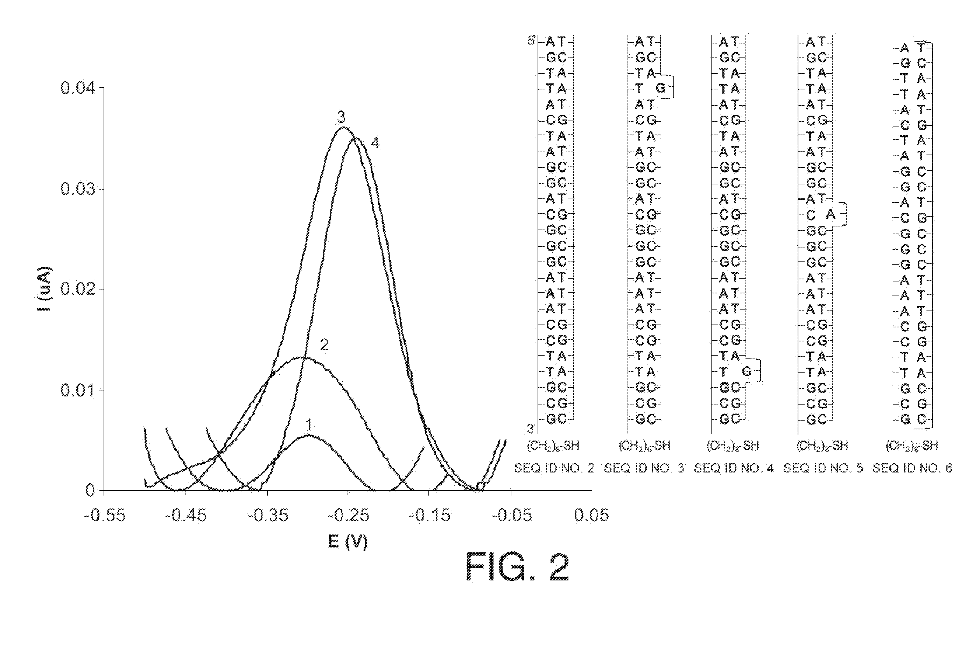

Figure 2. Differential pulse voltammograms (DPVs) of RuL accumulated in a gold electrode modified with (SEQ ID NO. 1): after the hybridization with a mismatched sequence in a base in the medium (SEQ ID NO. 4) (2) after the hybridization with a mismatched sequence (close to the 5' end of the sequence, (SEQ ID NO. 5), (3) after the hybridization with a mismatched sequence close to the 3' end of the sequence, (SEQ ID NO. 6), (4) and after the hybridization with a non-complementary sequence (SEQ ID NO. 3) (1).

### Object of the Invention

A new compound derived from ruthenium of formula (I), known as pentaamin ruthenium [3-(2-phenanthren-9-yl-vinyl)pyridine] complex is an object of the present invention.

A method for the detection of hybridization between a probe and a target nucleic acid sequence based on the use of said complex of formula I as an indicator of the hybridization is also an object of the invention.

Finally, the use of the compound derived from ruthenium of formula (I) as an electrochemical indicator of hybridization between nucleic acid sequences is an object of the present invention.

### Detailed Description of the Invention

The authors of the present invention have obtained, after considerable research labor, a new compound derived from ruthenium the structure of which allows the use thereof as an indicator of the process of hybridization between nucleic acid sequences in electrochemical biosensors for the detection of the mismatch of a single base, as well as the position thereof within the sequence.

Thus, a main aspect of the invention relates to a new compound derived from ruthenium of formula (I):

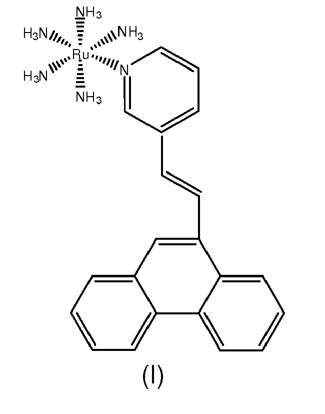

This new complex, known as pentaamin ruthenium [3-(2-phenanthren-9-yl-vinyl)pyridine] (referred to as RuL), prepared *in situ,* has a dual functional as a result of its structure. On one hand, the planar structure of the aromatic groups of the ligand 3-(2-phenanthren-9-yl-vinyl) pyridine (L) confers an intercalative character to the complex and therefore allows it to bind to double-stranded nucleic acids. This property differentiates it from the complexes used in the state of the art, the main form of binding to DNA of which is electrostatic (a much less specific binding than that occurring through intercalation), which gives rise to a more specific binding to DNA, allowing obtaining better results. On the other hand, the redox center of the metal (Ru) serves as an electrochemical indicator for detecting the hybridization event.

Based on the advantages derived from this new compound, a method for the electrochemical detection of the process of hybridization between nucleic acid sequences has been developed, based on the use of said compound as an indicator of the hybridization, which allows the detection of a single mismatched base, and the position thereof in the nucleotide sequence.

Thus, another main aspect of the invention contemplates a method for the detection of hybridization between a probe and a target nucleic acid sequence comprising the following phases:
a) immobilizing the nucleic acid probe on the surface of an electrode,
b) incubating the electrode, modified with the nucleic acid probe, with a solution susceptible of comprising the target sequence so that the hybridization takes place,
c) submersing the electrode after the incubation in a solution comprising the compound derived from ruthenium of formula (I),
d) applying cyclic potential scans to incorporate the compound derived from ruthenium to the double strand of nucleic acid formed in the hybridization, and
e) electrochemically detecting the hybridization between the probe and the target nucleic acid sequence.

The detection of the process of hybridization allows the detection of specific nucleic acid sequences, the quantification of nucleic acid sequences, the detection of a mismatch in a nucleic acid sequence or the detection of the position of said mismatch.

In the present invention, "probe" is defined as a nucleic acid fragment having a specificity of hybridization in certain conditions to hybridize with a target nucleic acid.

In a preferred embodiment "nucleic acid" is defined as DNA and/or RNA fragments, based on which the hybridization can be detected between DNA-DNA, DNA-RNA and RNA-RNA sequences.

As has been previously mentioned, the ruthenium complex of formula (I), due to the aromatic rings of the ligand (L), is preferably intercalated between the stacked bases of the double strand of nucleic acid, immobilized on the surface of the electrode, formed after the hybridization between the immobilized probe sequence and the target sequence present in the solution.

When a suitable potential is applied to said electrode, the oxidation of the redox center of ruthenium gives rise to a faradaic current directly related to the hybridization event. This allows detecting specific nucleic acid sequences (target sequences).

Furthermore, the intensity of the faradaic current increases proportionally to the extent of the hybridization, which allows the quantification of nucleic acid sequences.

Finally, the sensitivity and specificity obtained with this method, as a result of the use of the RuL compound as an indicator of the hybridization, allow the detection of mismatches in a single base of a certain sequence, as well as the position of said mismatches within the sequence.

In a preferred embodiment, the nucleic acid, both of the probe and of the target sequence, is DNA.

The electrode used in the described method can be of different types (carbon, gold, etc). In a particular embodiment, the electrode used is of gold.

The incorporation of the ruthenium complex (RuL) to the double strand is carried out by means of potential scans, which provides a higher sensitivity to the method, in comparison with those methods carrying out the incorporation at open circuit, in which there is no control over the incorporation of the material.

In a preferred embodiment the cyclic potential scans are applied 200 times, between -0.5 and 0.1 V.

The hybridization is detected through the changes in the electroactivity of the RuL complex using Differential Pulse Voltammetry (DPV). This occurs at a potential of approximately -0.30 V, which makes this complex particularly attractive due to its low redox potential, preventing possible interferences of oxidations of other compounds.

In another preferred embodiment, the electrochemical detection is carried out at a scan rate of 10 Mv/s, a pulse amplitude of 50 mV and a pulse width of 0.2 s.

Finally, another main aspect of the invention contemplates the use of the RuL compound of formula (I) as an electrochemical indicator of hybridization between nucleic acid sequences.

In a particular embodiment, the use of the RuL compound as an indicator of hybridization allows the detection of a specific nucleic acid sequence (target sequence).

In another particular embodiment, the use of the RuL compound allows the quantification of nucleic acid sequences.

In another particular embodiment, the use of the RuL compound allows the detection of a mismatched base in a nucleic acid sequence.

In another particular embodiment, the use of the RuL compound allows the detection of the position of a mismatched base in a nucleic acid sequence.

In preferred embodiments, the nucleic acid is DNA.

The following examples below illustrate the present invention, but must not be considered as limiting the essential aspects of the object thereof, as they have been set forth in the sections of this description.

### Example 1

### Synthesis of the RuL complex

80 µl of 2 mM Ru (NH₃)₅(H₂O) were directly mixed in solution with 80 µl of 2 mM of the ligand, (L=3-(2-phenanthren-9-yl-vinyl)-pyridine). The ligand 3-(2-phenanthren-9-yl-vinyl)-pyridine was synthesized according to the following procedure: 15 ml of N,N,N',N'-tetramethylenediamine were purged with pre-purified dry nitrogen, 2.6093 g of 9-bromophenanthrene, 1.3 ml of 4-vinylpyridine, 24.2 mg of palladium acetate and 0.1118 mg of triphenylphosphine were added and the mixture was purged for 15 minutes. It was maintained under reflux drying with previously purified nitrogen for 40 hours. Then, 250 ml of 0.1 M HCl were added to the mixture, a bright yellow precipitate appearing. The precipitate was filtered and washed with EtOH and CH₂Cl₂- Subsequently, the precipitate was neutralized with H₂O/0.1 M NaOH and extracted with CH₂Cl₂. Then it was reprecipitated with HCl and the yellow solid was recrystallized with H₂O/NaOH, the basic form (light beige color) of the ligand being obtained. Ru(NH₃)₅(H₂O) was synthesized according to the procedure described by C.G. Kehuen and H. Taube (Kuehn, C. C.; Taube, H. Journal of the American Chemical Society, 1976, 98(3), 689-702).

### Development of the (bio)sensor

It was prepared by means of the immobilization of the probe DNA sequence on electrodes of gold by direct absorption for 1 hour at room temperature. The modified electrodes were submersed in distilled water for 30 minutes to eliminate possible non-absorbed DNA residues. Before the immobilization, the electrodes were polished and conditioned according to a previously described procedure (Widrig, C. A.; Chung, C.; Porter, M. D. J. Electroanal. Chem. 1991, 310, 335-359*)*. The microscopic area of the electrodes was 1.7 mm².

### Hybridization and intercalation of the complex

The gold electrodes modified with the DNA probe were incubated for 1 hour at 40ºC with complementary, non-complementary DNA sequences and sequences with a mismatch, all of them prepared in hybridization buffer (10 mM phosphate, pH 7.0, with 0.4 M NaCl). After the hybridization, the electrodes were submersed in a 20 µM solution of RuL in 5 Mm KNO₃ and the potential was cyclically scanned 200 times, between -0.5 and 0.1 V. The electrodes were rinsed with distilled water before their use.

### Electrochemical measurements

A three-terminal electrochemical cell, of in-house production, with an auxiliary platinum electrode and a calomel electrode of saturated NaCl, also of in-house production, was used as a reference electrode. The solutions were purged with N₂ before each electrochemical measurement and the nitrogen atmosphere was maintained during the entire measurement. KNO₃ was used as support electrolyte. The measurement parameters of the differential pulse voltammetry (DPV) were: scan rate: 10 Mv/s; pulse amplitude: 50 mV; pulse width: 0.2 s.

### Results: Novel application of the invention

The developed system, which serves as prototype for the detection of any sequence, was applied to the detection of specific sequences of *Helicobacter pylori.* A 25-mer thiolated synthetic sequence of this bacteria (SEQ ID NO. 1) was immobilized on a gold electrode (immobilization density: 9.0 pmol/cm²) through the thiol groups. In the first hybridization assay, the complementary sequence (SEQ ID NO. 2) and the non-complementary sequence (SEQ ID NO. 3) were used as target sequences. The immobilization of the probe sequence (SEQ ID NO. 1), the hybridizations and the current measurements were carried out according to the previously described procedures and conditions. Figure 1 shows the differential pulse voltammograms (DPVs) of 20 µM RuL accumulated in an electrode modified with (SEQ ID NO. 1) obtained before (2) and after the hybridization with the complementary strand (SEQ ID NO. 2) (1) and the non-complementary strand (SEQ ID NO. 3) (3) strand.

It was observed that after the hybridization of (SEQ ID NO. 1) with the complementary strand (SEQ ID NO. 2) in the surface of the biosensor, an increase in the peak current intensity of the voltammogram was obtained, due to the oxidation of the ruthenium of the complex intercalated in the double-strand. Based on the ratio of the surface coatings it was possible to estimate that 5 molecules of the ruthenium complex were included for every 25 DNA base pairs. However, when the hybridization was carried out with the non-complementary sequence (SEQ ID NO. 3), no change was observed in the current (compare curve 2 and curve 3 of Figure 1). Therefore, the changes caused in the peak currents and in the potential indicate that the complex (RuL) serves as an electrochemical indicator for detecting the process of hybridization between *Helicobacter pylori* DNA fragments and therefore the recognition of certain fragments present in a sample. Furthermore, the current intensity at -0.260 V increased proportionally to the extent of the hybridization, i.e., upon increasing the amount of the sequence immobilized in the electrode (probe). The variations between electrodes can be corrected by means of the graphic representation of the normalized current (i-i0/i0) versus the amount of the probe, in which i0 and i represent the current before and after the hybridization for a certain amount of complementary SEQ ID NO. 2. The normalized current (i-i0/i0) measured at -0.260 V showed an excellent linear correlation (linear correlation coefficient, r = 0.995) with the increase of the complementary sequence SEQ ID NO. 2 in the range from 106 to 708 pmol. The limit of detection, calculated as the signal corresponding to the probe sequence (SEQ ID NO. 1) plus three times the standard deviation, was 92 ± 4 pmol of the complementary sequence (SEQ ID NO. 2) and the reproducibility of the determination was 94%.

### Detection of sequences with a mismatched base

The selectivity of the biosensor was evaluated by analyzing sequences containing a single mismatched base in different positions: in the middle (SEQ ID NO. 4) and at the ends (SEQ ID NO. 5) and (SEQ ID NO. 6), as indicated in the scheme of Figure 2, under the same hybridization conditions used for the completely complementary sequence (SEQ ID NO. 2). The distorted double helix formed after the hybridization of the probe (SEQ ID NO. 1) with the mismatched sequence in a base, accumulated a smaller amount of RuL and as a result, the current intensity obtained after the oxidation thereof was lower than that obtained after the hybridization with a completely complementary sequence (SEQ ID NO. 2). Figure 2 (curves 2, 3 and 4) shows the differential pulse voltammograms (DPVs) obtained with a gold electrode modified with the probe (SEQ ID NO. 1) after the hybridization with different sequences with a mismatch in different positions, using RuL as an electrochemical indicator. In all the cases there was a considerable reduction in the peak current comparing it with the response obtained when the probe SEQ ID NO. 1 hybridized with its completely complementary sequence (SEQ ID NO. 2) (Figure 1, curve 1). Furthermore, the current obtained when the mismatch was in the middle of the sequence was lower (see curve 2 of Figure 2) than that obtained when the mismatch was at the ends (Figure 2, curve 3 and 4 respectively), and was greater (50%) than that obtained with a completely non-complementary sequence (see curve 1 of Figure 2). This allowed unequivocally detecting the presence of a mismatch in a certain sequence and the position thereof.

In conclusion, the results clearly show the validity of the developed system, based on the use of the ruthenium complex (RuL) as an indicator of hybridization in an electrochemical biosensor for the detection of DNA sequences, for the quantification thereof and for the detection of a mismatched base and the position thereof.

## Claims

1. Compound derived from ruthenium of formula (I):

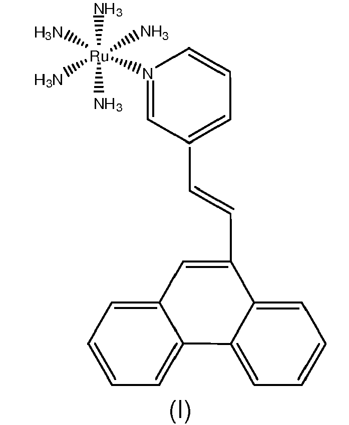

2. Method for the detection of hybridization between a probe and a target nucleic acid sequence, **characterized in that** it comprises the following steps:
a. immobilizing the nucleic acid probe on the surface of an electrode,
b. incubating the electrode, modified with the nucleic acid probe, with a solution susceptible of comprising the target sequence so that the hybridization takes place,
c. submersing the electrode, after the step b) of incubation in a solution comprising the compound derived from ruthenium of claim 1,
d. applying cyclic potential scans to incorporate the compound derived from ruthenium to the double strand of nucleic acid formed in the hybridization,
e. electrochemically detecting the hybridization between the
probe and the target nucleic acid sequence,
wherein the detection of hybridization allows the detection of a nucleic acid sequence, the quantification of nucleic acid sequences, the detection of a mismatched base in a nucleic acid sequence or the detection of the position of said mismatched base.

3. Method according to claim 2, **characterized in that** the nucleic acid of the probe and the target sequence is DNA.

4. Method according to claim 3, **characterized in that** the electrode used is of gold.

5. Method according to claim 4, **characterized in that** the cyclic potential scans are applied 200 times, between -0.5 and 0.1 V.

6. Method according to claim 4, **characterized in that** the electrochemical detection is carried out by differential pulse voltammetry at a scan rate of 10 Mv/s, a pulse amplitude of 50 mV and a pulse width of 0.2 s.

7. Use of the compound according to claim 1 as an electrochemical indicator of hybridization between nucleic acid sequences.

8. Use of the compound according to claim 7 for the detection of a nucleic acid sequence.

9. Use of the compound according to claim 7 for the quantification of nucleic acid sequences.

10. Use of the compound according to claim 7 for the detection of a mismatched base in a nucleic acid sequence.

11. Use of the compound according to claim 7 for the detection of the position of a mismatched base in a nucleic acid sequence.

12. Use of the compound according to any of claims 7-11, wherein the nucleic acid is DNA.


SEQUENCE LISTING

<110> UNIVERSIDAD AUTÓNOMA DE MADRID

<120> METHOD FOR THE ELECTROCHEMICAL DETECTION OF NUCLEIC
ACIDS SEQUENCES

<130> 116/07

<160> 6

<170> PatentIn version 3.4

<210> 1
<211> 25
<212> DNA
<213> Helicobacter pylori

<220>
<221> misc_feature
<223> Hexa alkylthiol molecule bound to the sequence at the 5'end
to immobilize the sequence on an electrode through the
thiol group.

<400> 1
gcgttccaaa gggcaggatc attga 25

<210> 2
<211> 25
<212> DNA
<213> Helicobacter pylori

<400>  2
tcaatgatcc tgccctttgg aacgc                                          25

<210>  3
<211>  25
<212>  DNA
<213>  Helicobacter pylori

<400>  3
gaccgtcgaa gtaaagggtt ccata                                          25

<210>  4
<211>  25
<212>  DNA
<213>  Helicobacter pylori

<400>  4
tcaatgatcc taccctttgg aagcg                                          25

<210>  5
<211>  25
<212>  DNA
<213>  Helicobacter pylori

<400>  5
tcagtgatcc tgccctttgg aacgc                                          25

<210>  6
<211>  25
<212>  DNA
<213>  Helicobacter pylori

<400>  6
tcaatgatcc tgccctttgg agcgc                                          25